# EUROPEAN PATENT APPLICATION

(11) **EP 4 008 317 A1**
(43) Date of publication of application: **08.06.2022**
(21) Application number: 21211906.9
(22) Date of filing: 02.12.2021
(51) Int. Cl.: A61K 9/20, A61K 9/28

(54) **SOLID PHARMACEUTICAL FORMULATIONS OF AMORPHOUS DAPAGLIFLOZIN**

(30) Priority: 03.12.2020 TR 202019592
(71) Applicant: SANOVEL ILAC SANAYI VE TICARET A.S., Istanbul 34460 (TR)
(72) Inventor: PEHLIVAN AKALIN, Nur, 34460 Istanbul (TR); SÜNEL, Fatih, 34460 Istanbul (TR); OZDEN, Aydan, 34460 Istanbul (TR); GÜLER, Tolga, 34460 Istanbul (TR)
(74) Representative: Sevinç, Erkan

(57) **Abstract**

The present invention relates to a solid pharmaceutical formulation comprising amorphous dapagliflozin and at least one pharmaceutically acceptable excipient. Further, the formulations comprising amorphous dapagliflozin are prepared a method which is simple, rapid, cost effective, time-saving and industrially convenient process.

## Description

### Field of the Invention

The present invention relates to a solid pharmaceutical formulation comprising amorphous dapagliflozin and at least one pharmaceutically acceptable excipient. Further, the formulations comprising amorphous dapagliflozin are prepared a method which is simple, rapid, cost effective, time-saving and industrially convenient process.

### Background of the Invention

Diabetes mellitus is a group of disorders of carbohydrate metabolism in which the action of insulin is diminished or absent through altered secretion, decreased insulin activity or a combination of both factors. There are two main types of diabetes; Type 1 and Type 2:
Type 1 diabetes occurs because the insulin-producing cells of the pancreas (beta cells) are damaged. In Type 1 diabetes, the pancreas makes little or no insulin, so sugar cannot get into the body's cells for use as energy. People with Type 1 diabetes must use insulin injections to control their blood glucose.

In Type 2 diabetes, the pancreas makes insulin, but it either doesn't produce enough, or the insulin does not work properly. This diabetes occurs most often in people who are over 40 years old and overweight and type 2 diabetes is the most common type, affecting more than 171 million people worldwide.Type 2 diabetes may sometimes be controlled with a combination of diet, weight management, and exercise. However, treatment also may include oral glucose-lowering medications or insulin injections.

Dapagliflozin is a sodium-glucose cotransporter 2 inhibitor (SGLT2). SGLT2 is a carrier responsible for the reabsorption of most of the glucose from the lumen of the renal tubule. SGLT2 is expressed in proximal renal tubules. By inhibiting SGLT2, dapagliflozin reduces the reabsorption of the filtered glucose and lowers the renal threshold for glucose. This improves urinary glucose excretion and blood glucose control. Dapagliflozin, also known as (1S)-1,5-Anhydro-1-C-[4-chloro-3-[(4-ethoxyphenyl)methyl]phenyl]-D-glucitol or (2S,3R,4R,5S,6R)-2-(3-(4-etoxybenzyl)-4-chlorophenyl)-6-hydroxymethyltetrahydro-2H-pyran-3,4,5-triol is represented by the structure of Formula I.

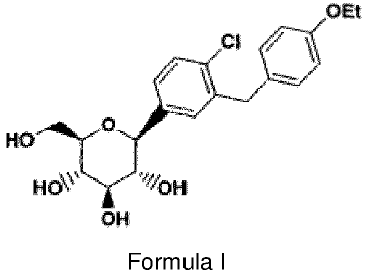

Dapagliflozin was first disclosed in patent US 6515117 (2003, Bristol-Myers Squibb).

US2008234366 A1, US2011064801 A1 and US2012263786 A1 disclose pharmaceutical compositions comprising dapagliflozin or dapagliflozin propylene glycol hydrate and specific excipients in given amounts.

WO2008116178 refers to pharmaceutical formulations which include crystalline dapagliflozin propylene glycol hydrate.

US2011064801 A1 further discloses pharmaceutical compositions in the form of a stock granulation comprising dapagliflozin propylene glycol hydrate and excipients in given amounts.

Although several solid forms comprising dapagliflozin are known in the art, finding a good or even the optimal form with regard to bioavailability and safety remains a considerable challenge, in particular when the compound forms many polymorphic forms. Not all solid forms comprising dapagliflozin are equally suitable with regard to stability, flow properties, compressibility, dissolution rate.

In view of the above art, there is still a need for an effective solid pharmaceutical formulation comprising of a form of dapagliflozin. The invention provides an amorphous form of dapagliflozin. Although amorphous forms are sometimes better soluble than crystalline forms, they are often not the preferred form because of water activity and/or stability reasons. However, the present invention eliminates all aforesaid problems and bring additional advantages to the relevant prior art.

### Detailed Description of the Invention

The main object of the present invention is to a solid pharmaceutical formulation comprising amorphous dapagliflozin which has the desired stability, dissolution profile, hardness and compressibility, in another words the disadvantages seen in the active substance in the art, is able to overcome.

Another object of the present invention is to eliminate problems and bringing additional advantages to the relevant prior art and problem of content uniformity which overcomes the above described problems in prior art.

The invention provides a solid pharmaceutical formulation comprising amorphous dapagliflozin. The solid pharmaceutical formulation does not comprise described problems in prior art about active agent. The formulation provides long shelf life and high stability during shelf life. Also, the solid pharmaceutical formulation comprising amorphous dapagliflozin provides the desired bioavailability.

According to an embodiment of the present invention, a solid pharmaceutical formulation comprising amorphous dapagliflozin and at least one pharmaceutically acceptable excipient.

As used here in, 'particle size' means the cumulative volume size distrubition as tested by any conventionally accepted method such as the laser diffraction method (i.e. malvern analysis). The term d (0.1) means, the size at which 10% by volume of the particles are finer and d (0.5) means the size at which 50% by volume of the particles are finer and d (0.9) means the size at which %90 by volume of the particles are finer.

We have found that dapagliflozin having the following particle sizes is very important for formulation. Especially, it positively affects the dissolution properties and powder homogenization. The obtained tablets have the desired dissolution profile. The powder is more homogeneous. The content uniformity of the tablets obtained from the more homogeneous powder is more ideal. Therefore, it provides better bioavailability.

According to an embodiment of the present invention, amorphous dapagliflozine has a d (0.9) particle size between 20 µm to 140 µm.

According to an embodiment of the present invention, amorphous dapagliflozine has a d (0.9) particle size between 22 µm to 130 µm, between 25 µm to 120 µm, between 30 µm to 110 µm, between 35 µm to 100 µm, between 40 µm to 98 µm, between 45 µm to 95 µm, between 47 µm to 90 µm, between 50 µm to 85 µm.

According to an embodiment of the present invention, amorphous dapagliflozine has a d (0.1) particle size between 3 µm to 60 µm.

According to an embodiment of the present invention, amorphous dapagliflozine has a d (0.1) particle size between 5 µm to 55 µm, between 6 µm to 50 µm, between 7 µm to 45 µm, between 8 µm to 40 µm, between 9 µm to 30 µm.

According to an embodiment of the present invention, amorphous dapagliflozine has a d (0.5) particle size between 8 µm to 90 µm.

According to an embodiment of the present invention, amorphous dapagliflozine has a d (0.5) particle size between 9 µm to 85 µm, between 10 µm to 80 µm, between 11 µm to 75 µm, between 11 µm to 70 µm, between 15 µm to 60 µm, between 18 µm to 55 µm, between 20 µm to 50 µm, between 25 µm to 45 µm.

According to an embodiment of the present invention, the amount of amorphous dapagliflozin is 0.05% to 8.0% by weight, preferably 0.05.% to 5.0% by weight in the total composition.

Dapagliflozin is used small proportion that can lead to considerable problems during the manufacture of the composition with regard to the uniformity of the content of active agent in the individual composition units. Because of problems uniformity of the content, the active substance may interact with several excipients. It reflects that content uniformity play important role in the dissolution of the drug. Using the right excipients is ensured uniformity of the content.

According to an embodiment of the present invention, at least one pharmaceutically acceptable excipient is selected from fillers, binders, disintegrants, lubricants, glidants or mixtures thereof.

Suitable fillers are selected from the group comprising microcrystalline cellulose, anhydrous lactose, lactose, starch, mannitol, calcium hydrogen phosphate dihydrate, dicalcium hydrogen phosphate anhydrate, calcium phosphate trihydrate, silicon dioxide, neutral pellets, magnesium carbonate, magnesium oxide, maltodextrin, maltose, medium chain triglycerides or mixtures thereof.

According to an embodiment of the present invention, the filler is microcrystalline cellulose or anhydrous lactose.

According to an embodiment of the present invention, the amount of fillers is 5.0% to 92% by weight.

According to an embodiment of the present invention, the amount of fillers is 5.0% to 40.0% or 58.0% to 92.0% by weight in the total composition.

Suitable binders are selected from the group comprising polyvinylpyrrolidone (povidone), hydroxypropylmethylcellulose, carboxymethylcellulose sodium, lactose anhydrous, pregelatinized starch, calcium carbonate, calcium phosphate dibasic, calcium phosphate tribasic, calcium sulphate, hydroxypropylcellulose, methyl cellulose, ethyl cellulose or mixtures thereof.

According to an embodiment of the present invention, the binder is polyvinylpyrrolidone or hydroxypropylmethylcellulose or carboxymethylcellulose sodium.

According to an embodiment of the present invention, the amount of binders is 0.5% to 15% by weight in the total composition. This amount of binders provides the desired dissolution profile.

Suitable disintegrants are selected from the group comprising croscarmellose sodium, crospovidone, sodium starch glycollate, sodium alginate, gums, starch and magnesium aluminum silicate or a mixture thereof.

According to an embodiment of the present invention, the disintegrant is croscarmellose sodium or crospovidone.

According to an embodiment of the present invention, the amount of disintegrants is 0.05% to 10.0% by weight, preferably 1.0% to 4.0% by weight in the total composition. This amount of disintegrants provides the desired dissolution profile.

Suitable glidant are selected from group comprising colloidal silicon dioxide, magnesium oxide, starch, magnesium silicate, colloidal silica or mixtures thereof.

According to an embodiment of the present invention, the glidant is colloidal silicon dioxide.

According to an embodiment of the present invention, the amount of glidants is 0.05% to 3.0% by weight in the total composition.

Suitable lubricants are selected from the group comprising magnesium stearate, sodium stearyl fumarate, silica colloidal anhydrous, talc, polyethylene glycol, stearic acid, aluminum silicate or mixtures thereof.

According to an embodiment of the present invention, the lubricant is magnesium stearate or sodium stearyl fumarate.

According to an embodiment of the present invention, the amount of lubricants is 0.01% to 5.0% by weight in the total composition.

According to an embodiment of the present invention, in the solid pharmaceutical formulation, modified release formulations can be preferred. Modified release formulations are selected from the group comprising controlled release, sustained release, delayed release, extended release, repeat action system or mixtures thereof. At this invention, formulation is prepared using matrix agents.

Suitable matrix agents are selected from the group comprising hydroxypropylmethyl cellulose, ethyl cellulose, polymethylmethacrylate derivatives's, poloxamer, polyvinyl alcohol, xanthan gum, sodium alginate, polymethacrylates, polyacrylamide, semi-synthetic polymers such as methyl cellulose, hydroxypropyl cellulose, polyvinyl acetate, cellulose acetate phthalate, ethylene vinyl acetate, methyl aminoethyl methacrylate, neutral methacrylic acid esters, polylactide, polylactide co-glycolide, low molecular weight polyethylene/polyisobutylene, polyanhydrides and poly (ortho-esters), diethyl aminoethyl methacrylate or mixtures thereof. The amount of matrix agents is 10.0% to 30.0% by weight in the total composition.

According to an embodiment of the present invention, the solid pharmaceutical formulation is in the form of film-coated tablet, bilayer tablet, multilayer tablet, orally disintegrating tablet, mini tablet, pellet, buccal tablet, sublingual tablet, effervescent tablet, capsule.

According to an embodiment of the present invention, the solid pharmaceutical formulation is in the form of film-coated tablet which comprises at least one film coating agent.

Suitable film coating agents are selected from the group comprising polymethacrylates, hydroxypropyl methylcellulose, lactose monohydrate, talc, hydroxypropyl cellulose, polyvinyl alcohol (PVA), polyethylene glycol (PEG), talc, glycerin, polyvinyl alcohol-polyethylene glycol copolymers (Kollicoat^{®} IR), ethylcellulose dispersions (Surelease^{®}), polyvinylprolidone, polyvinylprolidone-vinyl acetate copolymer (PVP-VA), iron oxide yellow, iron oxide, all kinds of Opadry^{®}, pigments, dyes, titanium dioxide, coloring agent or mixtures thereof.

According to an embodiment of the present invention, the film coating agents are selected from the group comprising polyvinyl alcohol, talc, titanium dioxide, polyethylene glycol, iron oxide, iron oxide yellow.

According to an embodiment of the present invention, the amount of film coating agents is 1.0% to 10.0% by weight, preferably 1.5 to 6.0% by weight in the total composition.

According to an embodiment of the present invention, the solid pharmaceutical formulation is in the form of bilayer tablet or multilayer tablet.

According to an embodiment of the present invention, amorphous dapagliflozin is present in a combination therapy. Especially; combinations can be with at least one dipeptidyl peptidase-4 (DPP-4) inhibitor agent. For example; amorphous dapagliflozin and metformin hydrochloride, amorphous dapagliflozin and saxagliptin in the form the free base or in the form of pharmaceutically acceptable salts, amorphous dapagliflozin and saxagliptin in the form the free base or in the form of pharmaceutically acceptable salts and metformin HCI, amorphous dapagliflozin and sitagliptin in the form the free base or in the form of pharmaceutically acceptable salts.

According to an embodiment of the present invention, amorphous dapagliflozin is present in a combination therapy which are with metformin HCl or saxagliptin in the form the free base or in the form of pharmaceutically acceptable salts or sitagliptin in the form the free base or in the form of pharmaceutically acceptable salts or mixtures thereof.

The solid pharmaceutical formulation of the present invention may be prepared, using standard techniques and manufacturing processes well known in the art, such as direct compression, wet granulation or dry granulation, hot melt granulation, hot melt extrusion, fluidized bed granulation, extrusion/spheronization, slugging, spray drying and solvent evaporation.

### Example 1: The film coated tablet

| **Active ingredient and excipient** | **Ingredients in one tablet (%) (by weight)** |
|---|---|
| Amorphous dapagliflozin | 0.05 - 8.0 |
| Microcrystalline cellulose | 50.0 - 80.0 |
| Anhydrous lactose | 8.0 - 25.0 |
| Crospovidone | 1.0 - 10.0 |
| Colloidal silicone dioxide | 0.5 - 5.0 |
| Magnesium stearate | 0.5 - 5.0 |
| Film coating agents | 1.0 - 8.0 |
| **Total** | **100** |

### Example 2: The film coated tablet

| **Active ingredient and excipient** | **Ingredients in one tablet (%) (by weight)** |
|---|---|
| Amorphous dapagliflozin | 3.8 |
| Microcrystalline cellulose | 66.8 |
| Anhydrous lactose | 19.2 |
| Crospovidone | 3.8 |
| Colloidal silicone dioxide | 1.5 |
| Magnesium stearate | 1.0 |
| Film coating agents | 3.8 |
| **Total** | **100** |

### A process for example 1 or 2;

a) Mixing amorphous dapagliflozin, microcrystalline cellulose, anhydrous lactose, crospovidone and colloidal silicone dioxide,
b) Adding magnesium stearate and then mixing,
c) Compressing the mixture to form of tablet,
d) Coating tablets with film coating agents.

### Example 3: The film coated tablet comprising amorphous dapagliflozin and metformin HCl

| **Active ingredient and excipient** | **Ingredients in one tablet (%) (by weight)** |
|---|---|
| Amorphous dapagliflozin | 0.05 - 5.0 |
| Metformin HCl | 55.0 - 80.0 |
| Polyvinylpyrrolidone | 2.0 - 15.0 |
| Microcrystalline cellulose | 5.0 - 30.0 |
| Croscarmellose sodium | 1.0 - 10.0 |
| Magnesium stearate | 0.1 -5.0 |
| Film coating agents | 2.0 - 10.0 |
| **Total** | **100** |

### Example 4: The film coated tablet comprising amorphous dapagliflozin and metformin HCl

| **Active ingredient and excipient** | **Ingredients in one tablet (%) (by weight)** |
|---|---|
| Amorphous dapagliflozin | 0.3 |
| Metformin HCI | 68.5 |
| Polyvinylpyrrolidone | 5.5 |
| Microcrystalline cellulose | 16.8 |
| Croscarmellose sodium | 2.4 |
| Magnesium stearate | 1.0 |
| Film coating agents | 5.5 |
| **Total** | **100** |

### A process for example 3 or 4;

a) Mixing amorphous dapagliflozin, metformin HC, croscarmellose sodium, microcrystalline cellulose,
b) Dissolving polyvinylpyrrolidone in pure water to obtained a solution,
c) Spraying the solution with the mixture at step (a).
d) Drying the mixture, then sieving the mixture,
e) Adding magnesium stearate and then mixing,
f) Compressing the mixture to form of tablet,
g) Coating tablets with film coating agents.

### Example 5: The bilayer tablet having extended release form comprising amorphous dapagliflozin and metformin HCl

| **Active ingredient and excipient** | **Ingredients in one layer (%) (by weight)** |
|---|---|
| **The immediate release layer (first layer)** | |
| Amorphous dapagliflozin | 0.1 -8.0 |
| Microcrystalline cellulose | 5.0 - 30.0 |
| Anhydrous lactose | 1.0 - 10.0 |
| Croscarmellose sodium | 0.05 - 3.0 |
| Colloidal silicon dioxide | 0.05 - 3.0 |
| Sodium stearyl fumarate | 0.05 - 3.0 |
| **The extended release layer (second layer)** | |
| Metformin HCI | 40.0 - 80.0 |
| At least one matrix agent (for example; hydroxypropyl methylcellulose (HPMC K100 MCR/K100/CN10T)) | 10.0 - 30.0 |
| Hydroxypropylmethyl cellulose as binder | 0.1 - 6.0 |
| Carboxymethylcellulose sodium | 0.5 - 6.0 |
| Magnesium stearate | 0.01 - 3.0 |

| **Film-coating** | |
|---|---|
| Film coating agents | 1.0 - 8.0 |
| **Total of the weight of the bilayer tablet** | **100** |

### Example 6: The bilayer tablet having extended release form comprising amorphous dapagliflozin and metformin HCl

| **Active ingredient and excipient** | **Ingredients in one layer (%) (by weight)** |
|---|---|
| **The immediate release layer (first layer)** | |
| Amorphous dapagliflozin | 0.6 |
| Microcrystalline cellulose | 11.7 |
| Anhydrous lactose | 2.9 |
| Croscarmellose sodium | 0.9 |
| Colloidal silicon dioxide | 0.3 |
| Sodium stearyl fumarate | 0.4 |

| **The extended release layer (second layer)** | |
|---|---|
| Metformin HCI | 59.9 |
| At least one matrix agent | 16.4 |
| Hydroxypropylmethylcellulose as binder | 1.2 |
| Carboxymethylcellulose Sodium | 3.0 |
| Magnesium stearate | 0.4 |

| **Film-coating** | |
|---|---|
| Film coating agents | 2.4 |
| **Total of the weight of the bilayer tablet** | **100** |

### A process for example 5 or 6;

**1.** The immediate release layer
   a. Mixing amorphous dapagliflozin and colloidal silicon dioxide,
   b. Adding anhydrous lactose and croscarmellose sodium,
   c. Adding microcrystalline cellulose and then mixing,
   d. Adding sodium stearyl fumarate and then mixing,
**2.** The extended release layer
   a. Mixing metformin HCI, carboxymethylcellulose sodium, hydroxypropyl methylcellulose
   b. Spraying the mixture with pure water,
   c. Drying the mixture, then sieving the mixture,
   d. Adding hydroxypropylmethylcellulose (HPMC K100 MCR/K100/CN10T) and then mixing,
   e. Adding magnesium stearate and then mixing,
**3.** Compressing the immediate release layer and extended release layer to form of a bilayer tablet,
**4.** Coating bilayer tablets with film coating agents.

## Claims

1. A solid pharmaceutical formulation comprising amorphous dapagliflozin and at least one pharmaceutically acceptable excipient.

2. The solid pharmaceutical formulation according to claim 1, wherein amorphous dapagliflozine has a d (0.9) particle size between 20 µm to 140 µm.

3. The solid pharmaceutical formulation according to claim 1, wherein amorphous dapagliflozine has a d (0.1) particle size between 3 µm to 60 µm.

4. The solid pharmaceutical formulation according to claim 1, wherein amorphous dapagliflozine has a d (0.5) particle size between 8 µm to 90 µm.

5. The solid pharmaceutical formulation according to claim 1, wherein the amount of amorphous dapagliflozin is 0.05% to 8.0% by weight, preferably 0.05.% to 5.0% by weight in the total composition.

6. The solid pharmaceutical formulation according to claim 1, wherein at least one pharmaceutically acceptable excipient is selected from fillers, binders, disintegrants, lubricants, glidants or mixtures thereof.

7. The solid pharmaceutical formulation according to claim 6, wherein fillers are selected from the group comprising microcrystalline cellulose, anhydrous lactose, lactose, starch, mannitol, calcium hydrogen phosphate dihydrate, dicalcium hydrogen phosphate anhydrate, calcium phosphate trihydrate, silicon dioxide, neutral pellets, magnesium carbonate, magnesium oxide, maltodextrin, maltose, medium chain triglycerides or mixtures thereof.

8. The solid pharmaceutical formulation according to claim 6, wherein binders are selected from the group comprising polyvinylpyrrolidone (povidone), hydroxypropylmethylcellulose, carboxymethylcellulose sodium, lactose anhydrous, pregelatinized starch, calcium carbonate, calcium phosphate dibasic, calcium phosphate tribasic, calcium sulphate, hydroxypropylcellulose, methyl cellulose, ethyl cellulose or mixtures thereof.

9. The solid pharmaceutical formulation according to claim 8, wherein the amount of binders is 0.5% to 15% by weight in the total composition.

10. The solid pharmaceutical formulation according to claim 6, wherein disintegrants are selected from the group comprising croscarmellose sodium, crospovidone, sodium starch glycollate, sodium alginate, gums, starch and magnesium aluminum silicate or a mixture thereof.

11. The solid pharmaceutical formulation according to claim 10, wherein the amount of disintegrants is 0.05% to 10.0% by weight in the total composition.

12. The solid pharmaceutical formulation according to claim 6, wherein glidant are selected from group comprising colloidal silicon dioxide, magnesium oxide, starch, magnesium silicate, colloidal silica or mixtures thereof.

13. The solid pharmaceutical formulation according to claim 6, wherein lubricants are selected from the group comprising magnesium stearate, sodium stearyl fumarate, silica colloidal anhydrous, talc, polyethylene glycol, stearic acid, aluminum silicate or mixtures thereof.

14. The solid pharmaceutical formulation according to claim 1, wherein the formulation is in the form of film-coated tablet, bilayer tablet, multilayer tablet, orally disintegrating tablet, mini tablet, pellet, buccal tablet, sublingual tablet, effervescent tablet, immediate release tablet, modified release tablet, capsule.

15. The solid pharmaceutical formulation according to claim 1, wherein amorphous dapagliflozin is present in a combination therapy which are with metformin HCI or saxagliptin in the form the free base or in the form of pharmaceutically acceptable salts or sitagliptin in the form the free base or in the form of pharmaceutically acceptable salts or mixtures thereof.
